# EUROPÄISCHE PATENTSCHRIFT

(11) **EP 1 687 013 B1**
(45) Veröffentlichungstag und Bekanntmachung des Hinweises auf die Patenterteilung: **14.02.2007**
(21) Anmeldenummer: 04738569.5
(22) Anmeldetag: 25.05.2004
(51) Int. Cl.: A61K 36/00

(54) **ARZNEIMITTEL, ENTHALTEND PRUNUS ARMENICA, COCOS NUCIFERA UND HUMULUS LUPULUS& x9;& x9;& x9;& x9;& x9;& x9;**
DRUG, COMPRISING PRUNUS ARMENICA, COCOS NUCIFERA AND HUMULUS LUPULUS
MEDICAMENT, RENFERMANT DU PRUNUS ARMENICA, COCOS NUCIFERA ET DE L'HUMULUS LUPULUS

(30) Priorität: 13.04.2004 DE 202004005959 U
(43) Veröffentlichungstag der Anmeldung: 09.08.2006
(73) Patentinhaber: Greifenstein, Peter, 83064 Raubling (DE)
(72) Erfinder: Greifenstein, Peter, 83064 Raubling (DE)
(86) Internationale Anmeldenummer: PCT/DE2004/001100
(87) Internationale Veröffentlichungsnummer: WO 2005/099727

(56) Entgegenhaltungen:
- GB-A- 417 513
- DATABASE MEDLINE [Online] US NATIONAL LIBRARY OF MEDICINE (NLM), BETHESDA, MD, US; Dezember 1992 (1992-12), YAMAMOTO K ET AL: "[Antimutagenic substances in the Armeniacae semen and Persicae semen]" XP002313369 Database accession no. NLM1294719 & YAKUGAKU ZASSHI. JOURNAL OF THE PHARMACEUTICAL SOCIETY OF JAPAN. DEC 1992, Bd. 112, Nr. 12, Dezember 1992 (1992-12), Seiten 934-939, ISSN: 0031-6903
- MANTI GUHA; SHYAMALI BASURAY; A. KUMAR SINHA: "Preventive effect of ripe banana in the diet on Ehrlich's ascitic carcinoma cell induced malignant ascites in mice" NUTRITION RESEARCH, Bd. 23, Nr. 8, August 2003 (2003-08), Seiten 1081-1088, XP002313367
- NAMASIVAYAM NALINI, VAIYAPURI MANJU, VENUGOPAL P. MENON: "Effect of coconut cake on the bacterial enzyme activity in 1,2-dimethyl hydrazine induced colon cancer" CLINICA CHIMICA ACTA, Bd. 342, Nr. 1-2, April 2004 (2004-04), Seiten 203-210, XP002313368

## Beschreibung

Die vorliegende Erfindung betrifft ein phytopharmakologisches Arzneimittel zumindest zur Behandlung des erworbenen Immundefekt-Syndroms (AIDS), von Krebs und von Erkrankungen der Psyche oder des Nervensystems, mit den im Oberbegriff des Patentanspruchs 1 angegebenen Merkmalen.

Aus dem Stand der Technik sind mehrere Arzneimittel zur Behandlung von AIDS, Krebs oder neurologischen Erkrankungen bekannt, welche einerseits mit erheblichen Herstellungskosten und andererseits mit einem beschränkten Wirkungsspektrum verbunden sind.

Aufgabe der vorliegenden Erfindung ist daher die Bereitstellung eines Arzneimittels, welches unter Aufwendung äußerst geringer finanzieller Mittel einfach und schnell herstellbar ist und über ein sehr breites Wirkungsspektrum verfügt.

Erfindungsgemäß wird diese Aufgabe bei einem gattungsgemäßen Arzneimittel durch die im kennzeichnenden Teil des Patentanspruchs 1 angegebenen Merkmale gelöst.

Besonders bevorzugte Ausführungsformen sind Gegenstand der Unteransprüche.

Im Rahmen der vorliegenden Erfindung wird ein Arzneimittel bereitgestellt, welches einen Gehalt an Bestandteilen und/oder an Extrakten von Prunus armenica und von Cocos nucifera und von Humulus lupulus und von gekeimter Gerste oder gekeimtem Roggen oder gekeimtem Weizen und von Myceten und von aus dem Traubensaft der Weinrebe durch alkoholische Gärung gewonnener Flüssigkeit und von Musazeen und von Blättern von Rubus, jeweils als Wirkstoff umfaßt.

Ein besonderer Vorteil des diese Wirkstoffkomponenten umfassenden erfindungsgemäßen Arzneimittels besteht darin, daß diese Wirkstoffkomponenten in großen Mengen und zu ausgesprochen günstigen Preisen auf dem Weltmarkt verfüg bar und erhältlich sind.

Erfindungsgemäß kann es sich bei den zum Einsatz kommenden Bestandteilen der Wirkstoffkomponenten beispielsweise um deren Fruchtfleisch, Fruchtstände, Saft, Milch, Kerne, Fasern, Zellfäden, Myzelien, Endosperm, Blätter, Blüten, Knospen, Schalen oder Stiele handeln.

So kann das erfindungsgemäße Arzneimittel beispielsweise einen Gehalt an Fruchtfleisch oder Kernen von Prunus armenica und an Fasern oder Endosperm von Cocos nucifera und an Fruchtständen von Humulus lupulus und an gekeimter Gerste oder gekeimtem Hafer oder gekeimtem Roggen oder gekeimtem Weizen und an Zellfäden oder Myzelien von Myceten und an aus dem Traubensaft der Weinrebe durch alkoholische Gärung gewonnener Flüssigkeit und an Früchten oder Schalen von Musazeen und an Blättern von Rubus, jeweils als Wirkstoff, aufweisen.

In einer bevorzugten Ausführungsform umfaßt das erfindungsgemäße Arzneimittel zusätzlich zu den obengenannten Bestandteilen oder Extrakten der Wirkstoffkomponenten ein oder mehrere übliche Trägerstoffe, Hilfsmittel und/oder Zusatzstoffe und kann dann beispielsweise in Form von einer Tablette oder eines Dragees oder eines Suppositoriums oder von Tropfen ausgebildet sein.

Wie bereits ausgeführt, kann das erfindungsgemäße Arzneimittel anstelle von oder zusätzlich zu den festen Bestandteilen der Wirkstoffkomponenten einen oder mehrere Extrakte aus den den Wirkstoffpflanzen oder Wirkstoffpfla nzenbestandteilen umfassen.

Dieses Exktrakt kann beispielsweise durch Fest-flüssig- oder Flüssig-flüssig-Extraktion der einzelnen Komponenten oder des gesamten Wirkstoff-Komponenten-Gemisches mit Hilfe eines üblichen Extraktionsmittels und durch anschließendes teilweises oder völliges Eindampfen der Extraktionslösu ng erhältlich sein.

Bei der erfindungsgemäß zum Einsatz kommenden Extraktion kann es sich beispielsweise um eine Heiß- oder Kaltextraktion sowie um ein kontinuierliches oder diskontinuierliches Extraktionsverfahren handeln.

Vorzugsweise handelt es sich bei dem erfindungsgemäß zum Einsatz kommenden kontinuierlichen Extraktionsverfahren um eine Soxhlet-Extraktion, eine Perforation oder um eine Perkolation.

Bei dem erfindungsgemäß zum Einsatz kommenden diskontinuierlichen Extraktionsverfahren kann es sich beispielsweise um ein Ausschütteln, Auslaugen, Auskochen oder Digerieren handeln.

In besonders bevorzugten Ausführungsformen des erfindungsgemäßen Arzneimittels stellt das Extrakt einen auf einen bestimmten Wirkstoffgehalt eingestellten Auszug aus einer einzelne n Wirkstoffkomponente oder aus dem gesamten Wirkstoffkomponentengernisch dar.
Dieser Auszug kann beispielsweise durch Mazeration oder Perkolation mittels Ethanol oder eines Ethanol/Wasser-Gemisches erhältlich sein.

Bei dem erfindungsgemäß zum Ei nsatz kommenden Extrakt kann es sich beispielsweise um ein Trockenextrakt (Extracta sicca) und/oder ein Fluidextrakt (Extracta fluida) und/oder ein zähflüssiges Extrakt (Extracta spissa) handeln.

Das erfindungsgemäße Arzneimittel kann beispielsweise in Form einer Flüssigkeit zur Einnahme in Form von Tropfen oder eines Aerosols oder in Form einer Lösung zur intravenösen, intraarteriellen, intramuskulären, subcutanen oder intralumbalen Injektion oder Infusion konfektioniert sein.

In besonders bevorzugten Ausführungsformen kann das erfindungsgemäße Arzneimittel einen Gehalt an Bestandteilen und/oder an Extrakten von Prunus armenica beispielsweise im Bereich von 10 Gewichts-% bis 20 Gewichts-% und von Cocos nucifera beispielsweise im Bereich von 10 Gewichts-% bis 20 Gewichts-% und von Humulus lupulus beispielsweise im Bereich von 10 Gewichts-% bis 20 Gewichts-% und von gekeimter Gerste oder gekeimtem Roggen oder gekeimtem Weizen beispielsweise im Bereich von 10 Gewichts-% bis 20 Gewichts-% und von Myceten beispielsweise im Bereich von 10 Gewichts-% bis 20 Gewichts-% und von aus dem Traubensaft der Weinrebe durch alkoholische Gärung gewonnener Flüssigkeit beispielsweise im Bereich von 10 Gewichts-% bis 20 Gewichts-% und von Musazeen beispielsweise im Bereich von 10 Gewichts-% bis 20 Gewichts-% und von Blättern von Rubus beispielsweise im Bereich von 10 Gewichts-% bis 20 Gewichts-% aufweisen, jeweils als Wirkstoff und gegebenenfalls neben den üblichen Trägerstoffen, Hilfsmitteln und/oder Zusatzstoffen.

Die vorstehend angeführten Bereiche von Gewichtsprozentangaben sind vor allem deshalb angezeigt, weil die Wirkstoffgehalte der einzelnen Wirkstoffkomponenten in Abhängigkeit von der Beschaffenheit des Aufzucht-Bodens jeweils zumindest etwas variieren.

Bei dem erfindungsgemäßen Arzneimittel kann die Wirkstoffkomponente "Myceten" beispielsweise ausgewählt sein aus der Gruppe, welche Chlorophyll-lose, eukaryontische Organismen umfaßt, insbesondere Protoctista (pilzähnliche Protisten) und/oder Fungi (höhere Pilze).

Insbesondere kann die erfindungsgemäße Wirkstoffkomponente "Myceten" ausgewählt sein aus der Gruppe, welche als Protoctista (niedere Pilze) Myxomycota, Myxomycetes, Acrasiomycetes, Plasmodiophoromycota, Labyrinthulomycota, Oomycota, Hyphochytriomycota und Chytridiomycota und als Fungi (höhere Pilze) Zygomycota, Ascomycota, Endomycetes, Ascomycetes, Basidiomycota, Ustomycetes, Basidiomycetes und Fungi imperfecti (Deuteromycetes) umfaßt.

Als "Myceten"-Wirkstoffkomponente können auch Bestandteile oder Extrakte von Dermatophyten, Hefen oder Schimmelpilzen erfindungsgemäß in Betracht kommen.

Bei der Wirkstoffkomponente "aus dem Traubensaft von Weinreben durch alkoholische Gärung gewonnene Flüssigkeit" kann es sich beispielsweise um Wein, vorzugsweise um Weißwein oder Rotwein, insbesondere der Rebsorten Trollinger oder Edelvernatsch, handeln.

Untersuchungen haben ergeben, daß das erfindungsgemäße Arzneimittel sowohl in fester als auch in flüssiger Form effektiv zur Behandlung unter anderem des erworbenen Immundefekt-Syndroms (AIDS) und/oder von Krebs, bösartigen Tumoren, Karzinomen oder Sarkomen und/oder von Erkrankungen der Psyche oder des Nervensystems geeignet ist.

Erfindungsgemäß sind die Bestandteile und/oder Extrakte von Prunus armenica und von Cocos nucifera und von Humulus lupulus und von gekeimter Gerste oder gekeimtem Roggen oder gekeimtem Weizen und von Myceten und von aus dem Traubensaft der Weinrebe durch alkoholische Gärung gewonnener Flüssigkeit und von Musazeen und von Blättern von Rubus zur Herstellung eines Arzneimittels zur Behandlung des erworbenen Immundefekt-Syndroms (AIDS) verwendbar.

Alternativ oder zusätzlich hierzu sind die Bestandteile und/oder Extrakte von Prunus armenica und von Cocos nucifera und von Humulus lupulus und von gekeimter Gerste oder gekeimtem Roggen oder gekeimtem Weizen und von Myceten und von aus dem Traubensaft der Weinrebe durch alkoholische Gärung gewonnener Flüssigkeit und von Musazeen und von Blättern von Rubus zur Herstellung eines Arzneimittels zur Behandlung von Krebs, bösartigen Tumoren, Karzinomen und Sarkomen verwendbar.

Alternativ oder zusätzlich hierzu sind die Bestandteile und/oder Extrakte von Prunus armenica und von Cocos nucifera und von Humulus lupulus und von gekeimter Gerste oder gekeimtem Roggen oder gekeimtem Weizen und von Myceten und von aus dem Traubensaft der Weinrebe durch alkoholische Gärung gewonnener Flüssigkeit und von Musazeen und von Blättern von Rubus zur Herstellung eines Arzneimittels zur Behandlung von Erkrankungen der Psyche und des Nervensystems verwendbar.

Darüberhinaus hat sich gezeigt, daß die Bestandteile und/oder Extrakte von Prunus armenica und von Cocos nucifera und von Humulus lupulus und von gekeimter Gerste oder gekeimtem Roggen oder gekeimtem Weizen und von Myceten und von aus dem Traubensaft der Weinrebe durch alkoholische Gärung gewonnener Flüssigkeit und von Musazeen und von Blättern von Rubus offensichtlich zur Herstellung eines Arzneimittels zur Behandlung von Diabetes-Erkrankungen verwendbar sind.

Zusammenfassend ist festzustellen, daß die vorliegende Erfindung aufgrund des Einsatzes besonders kostengünstig erhältlicher Wirkstoff-Komponenten und aufgrund der Herstellbarkeit des erfindung sgemäßen Arzneimittels mittels einfacher Herstellungsverfahren erstmals eine kostengünstige, einfache und schnelle Herstellung eines Arzneimittels mit einem sehr breiten Wirkungsspektrum zur Behandlung zumindest von AIDS, Krebs und psychischen Erkrankungen erlaubt.

## Patentansprüche

1. Arzneimittel, **gekennzeichnet durch** einen Gehalt an Bestandteilen und/oder an Extrakten von Prunus armenica und von Cocos nucifera und von Humulus lupulus und von gekeimter Gerste oder gekeimtem Roggen oder gekeimtem Weizen und von Myceten und von aus dem Traubensaft der Weinrebe **durch** alkoholische Gärung gewonnener Flüssigkeit und von Musazeen und von Blättern von Rubus, jeweils als Wirkstoff.

2. Arzneimittel nach Anspruch 1, **dadurch gekennzeichnet, daß** es sich bei den Bestandteilen der Wirkstoff-Komponenten gemäß Anspruch 1 um deren Fruchtfleisch, Fruchtstände, Saft, Milch, Kerne, Fasern, Zellfäden, Myzelien, Endosperm, Blätter, Blüten, Knospen, Schalen oder Stiele handelt.

3. Arzneimittel nach einem der vorhergehenden Ansprüche, **gekennzeichnet durch** einen Gehalt an Fruchtfleisch oder Kernen von Prunus armenica und an Fasern oder Endosperm von Cocos nucifera und an Fruchtständen von Humulus lupulus und an gekeimter Gerste oder gekeimtem Hafer oder gekeimtem Roggen oder gekeimtem Weizen und an Zellfäde n oder Myzelien von Myceten und an aus dem Traubensaft der Weinrebe **durch** alkoholische Gärung gewonnener Flüssigkeit und an Früchten oder Schalen von Musazeen und an Blättern von Rubus, jeweils als Wirkstoff.

4. Arzneimittel nach einem der vorhergehenden Ansprüche, **dadurch gekennzeichnet, daß** es zusätzlich zu den Bestandteilen und/oder Extrakten der Wirkstoffkomponenten gemäß Anspruch 1 übliche Trägerstoffe, Hilfsmittel und/oder Zusatzstoffe umfaßt und in Form von einer Tablette oder eines Dragees oder eines Suppositoriums oder von Tropfen ausgebild et ist.

5. Arzneimittel nach Anspruch 1, **dadurch gekennzeichnet, daß** das Extrakt durch Fest-flüssig- oder Flüssig-flüssig-Extraktion der einzelnen Komponenten oder des Wirkstoff-Komponenten-Gemisches mit Hilfe eines üblichen Extraktionsmittels und durch anschließendes teilweises oder völliges Eindampfen der Extraktionslösung erhältlich ist.

6. Arzneimittel nach einem der Ansprüche 1 oder 5, **dadurch gekennzeichnet, daß** es sich bei der Extraktion um eine Heiß- oder Kaltextraktion sowie um eine kontinuierliche oder diskontinuierliche Extraktion handelt.

7. Arzneimittel nach einem der Ansprüche 1, 5 oder 6, **dadurch gekennzeichnet, daß** das kontinuierliche Extraktionsverfahren eine Soxhlet-Extraktion, Perforation oder Perkolation ist, während es sich bei dem diskontinuierlichen Extraktionsverfahren um ein Ausschütteln, Auslau gen, Auskochen oder Digerieren handelt.

8. Arzneimittel nach einem der Ansprüche 1, 5, 6 oder 7, **dadurch gekennzeichnet, daß** das Extrakt einen auf einen bestimmten Wirkstoffgehalt eingestellten Auszug aus einer einzelnen Wirkstoffkomponente oder aus dem gesamten Wirkstoffkomponentengemisch darstellt, welcher durch Mazeration oder Perkolation mittels Ethanol oder Ethanol/Wasser-Gemischen erhältlich ist.

9. Arzneimittel nach einem der Ansprüche 1, 5, 6, 7 oder 8, **dadurch gekennzeichnet, daß** es sich bei dem Extrakt um ein Trockenextrakt (Extracta sicca) und/oder ein Fluidextrakt (Extracta fluida) und/oder ein zähflüssiges Extrakt (Extracta spissa) handelt.

10. Arzneimittel nach einem der Ansprüche 1, 5, 6, 7, 8 oder 9, **dadurch gekennzeichnet, daß** es in Form einer Flüssigkeit zur Einnahme in Form von Tropfen oder eines Aerosols oder in Form einer Lösung zur intravenösen, intraarteriellen, intramuskulären, subcutanen oder intralumbalen Injektion oder Infusion konfektioniert ist.

11. Arzneimittel nach einem der vorhergehenden Ansprüche, **dadurch gekennzeichnet, daß** es einen Gehalt an Bestandteilen und/oder an Extrakten von Prunus armenica im Bereich von 10 Gewichts-% bis 20 Gewichts-% und von Cocos nucifera im Bereich von 10 Gewichts-% bis 20 Gewichts-% und von Humulus lupulus im Bereich von 10 Gewichts-% bis 20 Gewichts-% und von gekeimter Gerste oder gekeimtem Roggen oder gekeimtem Weizen im Bereich von 10 Gewichts-% bis 20 Gewichts-% und von Myceten im Bereich von 10 Gewichts-% bis 20 Gewichts-% und von aus dem Traubensaft der Weinrebe durch alkoholische Gärung gewonnener Flüssigkeit im Bereich von 10 Gewichts-% bis 20 Gewichts-% und von Mu sazeen im Bereich von 10 Gewichts-% bis 20 Gewichts-% und von Blättern von Rubus im Bereich von 10 Gewichts-% bis 20 Gewichts-% aufweist, jeweils als Wirkstoff neben den üblichen Trägerstoffen, Hilfsmitteln und/oder Zusatzstoffen.

12. Arzneimittel nach einem der vorhergehenden Ansprüche, **dadurch gekennzeichnet, daß** die Wirkstoffkomponente Myceten ausgewählt ist aus der Gruppe, welche Chlorophyll-lose, eukaryontische Organismen umfaßt, beispielsweise Protoctista (pilzähnliche Protisten) und/oder Fungi (höhere Pilze) .

13. Arzneimittel nach einem der vorhergehenden Ansprüche, **dadurch gekennzeichnet, daß** es zur Behandlung des erworbenen Immundefekt-Syndroms (AIDS) und/oder von Krebs, bösartigen Tumoren, Karzinomen oder Sarkomen und/oder von Erkrankungen der Psyche oder des Nervensystems geeignet ist.

14. Verwendung von Bestandteilen und/oder Extrakten von Prunus armenica und von Cocos nucifera und von Humulus lupulus und von gekeimter Gerste oder gekeimtem Roggen oder gekeimtem Weizen und von Myceten und von aus dem Traubensaft der Weinrebe durch alkoholische Gärung gewonnener Flüssigkeit und von Musazeen und von Blättern von Rubus zur Herstellung eines Arzneimittels zur Behandlung des erworbenen Immundefekt-Syndroms (AIDS).

15. Verwendung von Bestandteilen und/oder Extrakten von Prunus armenica und von Cocos nucifera und von Humulus lupulus und von gekeimter Gerste oder gekeimtem Roggen oder gekeimtem Weizen und von Myceten und von aus dem Traubensaft der Weinrebe durch alkoholische Gärung gewonnener Flüssigkeit und von Musazeen und von Blättern von Rubus zur Herstellung eines Arzneimittels zur Behandlung von Krebs, bösartigen Tumoren, Karzinomen und Sarkomen.

16. Verwendung von Bestandteilen und/oder Extrakten von Prunus armenica und von Cocos nucifera und von Humulus lupulus und von gekeimter Gerste oder gekeimtem Roggen oder gekeimtem Weizen und von Myceten und von aus dem Traubensaft der Weinrebe durch alkoholische Gärung gewonnener Flüssigkeit und von Musazeen und von Blättern von Rubus zur Herstellung eines Arzneimittels zur Behandlung von Erkrankungen der Psyche und des Nervensystems.

## Claims

1. A medicinal product **characterized in that** through a content of components and/or extracts of prunus armenica and of cocos nucifera and of humulus lupulus and germinated barley or germinated rye, or germinated wheat and of mycete, and the liquid obtained from alcoholic fermentation of the grape juice of grapevines, and from musaze and from rubus leaves, in each case as active ingredient.

2. The medicinal product according to Claim 1, **characterized in that** it consists of the active ingredient components in accordance with Claim 1 and their pulp, multiple fruit, juice, milk, kernels, fibers, cell filaments, myzelles, endosperm, leaves, blossoms, buds, hulls or stalks.

3. The medicinal product according to one of the preceding Claims, **characterized in that** through a content of pulp or kernels of prunus armenica and fibers or endosperm of cocos nucifera and multiple fruit of humulus lupulus and germinated barley or germinated oats or germinated rye or germinated wheat and cell filaments or myzelles of mycetes, and the liquid obtained by alcoholic fermentation of grape juice from the grapevine and the fruits or hulls of musaze and rubus leaves, in each case as an active ingredient.

4. The medicinal product according to one of the preceding Claims, **characterized in that** in addition to the extracts and/or component extracts in accordance with Claim 1 it comprises common carrier materials, auxiliary means and/or additives and is made up in the form of a tablet or a sugar coated tablet or a suppository or drops.

5. The medicinal product according to Claim 1, **characterized in that** the extract can be obtained by solid-liquid or liquid-liquid extraction of the individual components or the active ingredient component mixture with the help of common extraction means, and by subsequent partial or complete evaporation of the extraction solution.

6. The medicinal product according to Claims 1 or 5, **characterized in that** it is a question of whether there is hot or cold extraction as well as whether the extraction method is continuous or discontinuous.

7. The medicinal product according to Claims 1, 5 or 6 **characterized in that** the continuous extraction method is a Soxhlet extraction, perforation or a percolation, while the the discontinuous extraction method can be a shaking out, leaching out or digestion.

8. The medicinal product according to Claims 1, 5, 6 or 7 **characterized in that** the extract represents one of one fixed active ingredient content adjusted extract from one individual active ingredient or from the entire mixture of active components, which can be obtainable by means of maceration or percolation using ethanol or an ethanol-water mixtures.

9. The medicinal product according to Claims 1, 5, 6 or 8 **characterized in that** we are dealing with a dry extract (extracta sicca) and/or a liquid extract (extracta fluidica) and/or a viscous extract (extracta spissa).

10. The medicinal product according to Claims 1, 5, 6, 8 or 9 **characterized in that** it can be compounded in the form of a liquid to be taken in the form of drops or an aerosol or in the form of a solution for intravenous, intraarterial, intramuscular, subcutaneous or intralumbar injection or infusion.

11. The medicinal product according to the preceding Claims **characterized in that** it can have a content of ingredients and/or extracts of prunus armenica in the range of 10 wt% to 20 wt% and of cocus nucifera in the range from 10 wt% to 20 wt% and of humulus lupulus in the range from 10 wt% to 20 wt% and of germinated barley in the range from 10 to 20 wt% and of germinated rye in the range from 10 wt% to 20 wt% or germinated wheat from 10 wt% to 20 wt% and from mycete in the range from 10 wt% to 20 wt% and the liquid obtained by alcoholic fermentation of grape juice of the grapevine for example in the range from 10 wt% to 20 wt% and of musazes for example in the range from 10 wt% to 20 wt% and of rubus leaves in the range from 10 wt% to 20 wt%, in each case as the active ingredient together with the usual carrier materials, auxiliary means and/or additives.

12. The medicinal product according to the preceding Claims **characterized in that** the active ingredients mycete is selected from the group, which compises chlorophyll-free, eukaryontic organisms, especially protoctista (fungus like protista) and/or fungi (higher fungi).

13. The medicinal product according to the preceding Claims **characterized in that** it is suitable for treatment of Acquired Immune Deficiency Syndrome (AIDS) and/or cancer, malignant tumors, carcinomas, sarcomas and/or diseases of the psyche or of the nervous system.

14. Use of components and/or extracts of prunus armenica and of cocos nucifera and of humulus lupulus and germinated barley or germinated rye, or germinated wheat and of mycete, and the liquid obtained from alcoholic fermentation of the grape juice of grapevines, and from musaze and from rubus leaves, for preparation of a medicinal product for treatment of Acquired Immune Deficiency Syndrome (AIDS).

15. Use of components and/or extracts of prunus armenica and of cocos nucifera and of humulus lupulus and germinated barley or germinated rye, or germinated wheat and of mycete and the liquid obtained from alcoholic fermentation of the grape juice of grapevines, and from musaze, and from rubus leaves for preparation of a medicinal product for treatment of cancer, malignant tumors, carcinomas and sarcomas.

16. Use of components and/or extracts of prunus armenica and of cocos nucifera and of humulus lupulus and germinated barley or germinated rye, or germinated wheat and of mycete and the liquid obtained from alcoholic fermentation of the grape juice of grapevines, and from musaze, and from rubus leaves for preparation of a medicinal product for treatment of diseases of the psyche or of the nervous system.

## Revendications

1. Médicament, **caractérisé par** une teneur en composants et/ou extraits de Prunus armenica et de Cocos nucifera et de Humulus lupulus et d'orge germée, de seigle germé ou de blé germé et de mycètes et de liquide extrait de jus de raisin de vigne par fermentation alcoolique, et de Musacum et de feuilles de Rubus, chaque fois en tant que principe actif.

2. Médicament selon la revendication 1, **caractérisé en ce que** les éléments des composants de principes actifs selon la revendication 1 sont leur pulpe, leur infrutescences, leur jus, leur lait, leur coma, leur fibres, leurs mitoses, leurs micelles, leur endosperme, leurs feuilles, leurs fleurs, leurs bourgeons, leurs écorces ou leurs tiges.

3. Médicament, selon l'une quelconque des revendications précédentes, **caractérisé par** une teneur en pulpe ou en pépins de Prunus armenica et en fibres ou endosperme de Cocos nucifera et en infrutescences de Humulus lupulus et en orge germée ou en avoine germée ou en seigle germé ou en blé germé ou en mitoses ou en micelles de mycètes et en liquide extrait du raisin de vigne par fermentation alcoolique et en fruits ou en écorces de Musacum et en feuilles de Rubus, chaque fois en tant que principe actif.

4. Médicament selon l'une quelconque des revendications précédentes, **caractérisé en ce qu'**en supplément des éléments et/ou extraits des composants des principes actifs selon la revendication 1, il comprend des substances porteuses, des adjuvants et/ou des additifs usuels et **en ce qu'**il est réalisé sous la forme d'un comprimé, d'une dragée ou d'un suppositoire ou de gouttes.

5. Médicament selon la revendication 1, **caractérisé en ce que** l'extrait peut être obtenu par extraction graisse/liquide ou liquide/graisse des composants individuels ou du mélange des composants du principe actif à l'aide d'un produit d'extraction usuel ou par concentration partielle ou totale consécutive par évaporation de la solution d'extraction.

6. Médicament selon l'une quelconque des revendications 1 ou 5, **caractérisé en ce que** l'extraction est une extraction à chaud ou à froid, ainsi qu'une extraction continue ou discontinue.

7. Médicament selon l'une quelconque des revendications 1, 5 ou 6, **caractérisé en ce que** le procédé d'extraction continue est une extraction Soxhlet, une perforation ou une percolation, alors que le procédé d'extraction discontinue est une extraction par solvant, par lessivage, par cuisson ou par digestion.

8. Médicament selon l'une quelconque des revendications 1, 5, 6 ou 7, **caractérisé en ce que** l'extrait est un extrait réglé à une teneur déterminée de principe actif d'un composant individuel du principe actif ou de l'ensemble du mélange des composants du principe actif, que l'on obtient par macération ou par percolation au moyen d'éthanol ou de mélanges éthanol/eau.

9. Médicament selon l'une quelconque des revendications 1, 5, 6, 7 ou 8, **caractérisé en ce que** l'extrait est un extrait sec (Extracta sicca) et/ou un extrait fluide Extracta fluida) et/ou un extrait visqueux (Extracta spissa).

10. Médicament selon l'une quelconque des revendications 1, 5, 6, 7, 8 ou 9, **caractérisé en ce qu'**il est confectionné sous la forme d'un liquide, à administrer sous forme de gouttes ou d'un aérosol, ou sous forme d'une solution, pour l'injection intraveineuse, intra-artérielle, intramusculaire, sous-cutanée, intralombaire ou par perfusion.

11. Médicament selon l'une quelconque des revendications précédentes, **caractérisé en ce qu'**il comporte une teneur en éléments et/ou en extraits de Prunus armenica de l'ordre de 10 % en poids à 20 % en poids et de Cocos nucifera de l'ordre de 10 % en poids à 20 % en poids et de Humulus lupulus, de l'ordre de 10 % en poids à 20 % en poids et d'orge germée ou de seigle germé ou de blé germé de l'ordre de 10 % en poids à 20 % en poids et de mycètes de l'ordre de 10 % en poids à 20 % en poids et de liquide extrait de jus de raisin de vigne par fermentation alcoolique de l'ordre de 10 % en poids à 20 % en poids et de Musacum de l'ordre de 10 % en poids à 20 % en poids et de feuilles de Rubus de l'ordre de 10 % en poids à 20 % en poids, chaque fois en tant que principe actif, parallèlement aux substances porteuses, adjuvants et/ou additifs usuels.

12. Médicament selon l'une quelconque des revendications précédentes, **caractérisé en ce que** les composants du principe actif mycètes sont choisis dans le groupe comprenant la chlorophyllose, les organismes eucaryotes, par exemple Protoctista (protistes similaires à des champignons) et/ou Fungi (champignons supérieurs).

13. Médicament selon l'une quelconque des revendications précédentes, **caractérisé en ce qu'**il est adapté pour le traitement du syndrome immunodéficitaire acquis (SIDA) et/ou du cancer, de tumeurs malignes, de carcinomes ou de sarcomes et/ou d'affections psychiques ou du système nerveux.

14. Utilisation de composants et/ou extraits de Prunus armenica et de Cocos nucifera et de Humulus lupulus et d'orge germée ou de seigle germé ou de blé germé et de mycètes et de liquide extrait de jus de raisin de vigne par fermentation alcoolique, et de Musacum et de feuilles de Rubus pour la fabrication d'un médicament pour le traitement du syndrome immunodéficitaire acquis (SIDA).

15. Utilisation de composants et/ou extraits de Prunus armenica et de Cocos nucifera et de Humulus lupulus et d'orge germée ou de seigle germé ou de blé germé et de mycètes et de liquide extrait de jus de raisin de vigne par fermentation alcoolique, et de Musacum et de feuilles de Rubus pour la fabrication d'un médicament pour le traitement du cancer, de tumeurs malignes, de carcinomes et de sarcomes.

16. Utilisation de composants et/ou extraits de Prunus armenica et de Cocos nucifera et de Humulus lupulus et d'orge germée ou de seigle germé ou de blé germé et de mycètes et de liquide extrait de jus de raisin de vigne par fermentation alcoolique, et de Musacum et de feuilles de Rubus pour la fabrication d'un médicament pour le traitement d'affections psychologiques ou du système nerveux.
